# EUROPÄISCHE PATENTSCHRIFT

(11) **EP 4 228 744 B1**
(45) Veröffentlichungstag und Bekanntmachung des Hinweises auf die Patenterteilung: **23.07.2025**
(21) Anmeldenummer: 20800777.3
(22) Anmeldetag: 13.10.2020
(51) Int. Cl.: A61N 5/06

(54) **BESTRAHLUNGSVORRICHTUNG UND SYSTEM ZUR PHOTODYNAMISCHEN DESINFEKTION VON ZÄHNEN UND ZAHNFLEISCH**
IRRADIATION DEVICE AND SYSTEM FOR PHOTODYNAMIC DISINFECTION OF TEETH AND GUMS
DISPOSITIF D'IRRADIATION ET SYSTÈME DE DÉSINFECTION PHOTODYNAMIQUE DES DENTS ET DES GENCIVES

(43) Veröffentlichungstag der Anmeldung: 23.08.2023
(73) Patentinhaber: Mana Health Technologies GmbH, 8047 Graz (AT)
(72) Erfinder: SMOLLE, Johannes, 8047 Graz (AT); BÖHM, Elisa, 8047 Graz (AT)
(74) Vertreter: Weiser Voith Gugler Patentanwälte Partnerschaft
(86) Internationale Anmeldenummer: PCT/AT2020/060366
(87) Internationale Veröffentlichungsnummer: WO 2022/077037

(56) Entgegenhaltungen:
- WO-A1-2021/069805
- US-A1- 2008 032 252
- US-A1- 2008 255 498
- US-A1- 2016 051 833
- US-A1- 2018 256 916

## Beschreibung

Die vorliegende Erfindung betrifft eine Bestrahlungsvorrichtung zur photodynamischen Desinfektion von Zähnen und Zahnfleisch in einem Mund. Die Erfindung betrifft ferner ein System, welches eine derartige Bestrahlungsvorrichtung umfasst.

Bei der photodynamischen Desinfektion von Zähnen und Zahnfleisch im Mund wird im Zusammenspiel von Licht und einem Photosensibilisator (auch "Photosensitizer" genannt) im Mund ein meist durch Bakterien gebildeter schädlicher Biofilm an den Zähnen und am Zahnfleisch vernichtet und dadurch z.B. Karies, Gingivitis und/oder Parodontitis bekämpft. Der Photosensibilisator dringt in die Bakterien ein bzw. legt sich an sie an und wird durch die Bestrahlung aktiviert. Dabei werden freie Radikale, z.B. reaktive Sauerstoffspezies ("ROS"), erzeugt. Die freien Radikale sind weder krebserregend noch für den Menschen genotoxisch und wirken als Biozid, d.h. als eine Substanz, welche Krankheitserreger, z.B. Pilze (inklusive deren Sporen), Bakterien, Parasiten und Viren zerstört. Je nach Art des Photosensibilisators wirkt dieser bei der photodynamischen Desinfektion entweder als Katalysator, welcher bei Belichtung die freien Radikale aus der Umgebung erzeugt, z.B. aus einer den Photosensibilisator enthaltenden Lösung; oder der Photosensibilisator gibt bei Bestrahlung selbst freie Radikale ab. Die Wellenlänge der von der Strahlungsquelle abzugebenen Strahlung ist mit dem verwendeten Photosensibilisator und seinem Absorptionsspektrum abzustimmen; sie reicht im Allgmeinen vom Ultraviolett- bis in den Infrarot-Bereich.

Aus der US 2015/0044628 A1 ist ein Gerät zum Weißen von Zähnen bekannt, welches eine gekrümmte Rinne und einen davon vorspringenden Griff hat. Die Rinne wird mit einer photoaktivierbaren Substanz, z.B. einem Gel, gefüllt und wie ein Zahnabdrucklöffel über den Zahnbogen des Ober- oder Unterkiefers gedrückt. An ihren inneren Rinnen-Seitenwänden hat die Rinne Ultraviolett-LEDs zur Bestrahlung der Zähne. Nachteilig ist dabei einerseits, dass die Rinne infolge ihrer Größe und die Gelfüllung im Mund als äußerst unangenehm empfunden werden und z.B. oft einen Würgreiz auslösen. Auch sind für verschiedene Gebisse mehrere Rinnen unterschiedlicher Größe und/oder Krümmung bereitzustellen. Ferner wirkt die Ultraviolett-Bestrahlung lokal begrenzt, sodass eine Vielzahl von LEDs erforderlich ist, was einer Verkleinerung der Rinne im Wege steht und angesichts des mit kleinerer Wellenlänge sinkenden Wirkungsgrads der LEDs zu erhöhter Abwärme führt.

Aus der WO 2011/084744 A1 ist die Verwendung von Infrarot-LEDs zur photodynamischen Desinfektion eines Mundes bekannt. Um übermäßiger Erwärmung durch die Infrarot-Strahlung entgegenzuwirken, wird angeregt, nur Teile des Mundes punktuell zu bestrahlen.

Die US 2018/0256916 A1 zeigt eine Bestrahlungsvorrichtung zur photodynamischen Munddesinfektion mit einer außerhalb des Mundes liegenden Strahlungsquelle, deren Strahlung mit einem bogenförmigen Lichtleiter in den Mund geleitet wird.

Die Erfindung setzt sich zum Ziel, eine Bestrahlungsvorrichtung und ein System zu schaffen, welche einfach, komfortabel und effizient anwendbar sind.

Dieses Ziel wird gemäß einem ersten Aspekt der Erfindung durch eine Bestrahlungsvorrichtung zur photodynamischen Desinfektion von Zähnen und Zahnfleisch in einem Mund erreicht, umfassend ein in den Mund einführbares Mundstück, welches eine dem Zahnbogen nachgebildete U-Form hat und mit einer oder mehreren Strahlungsquellen für Strahlung in einem Wellenlängenbereich zwischen 500 nm und 1000 nm ausgestattet ist, ein Griffstück, welches vom Mundstück derart vorspringt, dass es nach Einführen des Mundstücks aus dem Mund herausragt, und eine Steuerung zum Ansteuern der Strahlungsquellen, wobei das Griffstück einen Kühlkörper aufweist und die Strahlungsquellen auf ein wärmeleitendes Substrat aufgebracht sind, welches die Strahlungsquellen mit dem Kühlkörper wärmeleitend verbindet.

Der Erfindung liegt die Erkenntnis zugrunde, dass Strahlung im Wellenlängenbereich zwischen 500 nm und 1000 nm auch im Zahnschmelz weitergeleitet und dabei von diesem abgestrahlt wird. Daraus resultiert einerseits, dass die Strahlung weniger lokal begrenzt wirkt als beispielsweise Ultraviolett-Strahlung, was eine kleinere Bauform der Bestrahlungsvorrichtung, insbesondere ihres Mundstücks, und in der Folge eine einfache und effiziente Anwendung bei gleicher Desinfektionswirkung ermöglicht. Um einer bei empfindlichen Zähnen unangenehmen Wärmeempfindung infolge der die Strahlung begleitenden Wärmewirkung vorzubeugen, ist andererseits eine wirkungsvolle Wärmeabfuhr über das wärmeleitende Substrat vorgesehen, wodurch die Bestrahlung besonders sicher und komfortabel ist.

In einer vorteilhaften Ausführungsform ist das Substrat eine in einer durch die U-Form aufgespannten Ebene liegende Metallkern-Leiterplatte. Die Metallkern-Leiterplatte erfüllt zweierlei Funktion: sie trägt und kontaktiert die Bauteile und leitet die Wärme der Strahlungsquellen ab. Ein separates wärmeleitendes Substrat kann dadurch entfallen, was einen einfachen Aufbau und eine geringe Baugröße - insbesondere des Mundstücks - ermöglicht.

In einer besonders bevorzugten Variante dieser Ausführungsform sind die Strahlungsquellen auf zumindest eine der Ober- und Unterseiten der Metallkern-Leiterplatte aufgebracht und haben zur genannten Ebene normale Strahlungsrichtungen. Das Mundstück kann dadurch besonders flach und platzsparend in Schichten aufgebaut sein. Durch den Zahnschmelz wird die Strahlung ausgehend von der Kaufläche der Backenzähne bzw. der Schneidkante der Schneidezähne entlang jedes Zahns bis in die Zahnfleischfurche zwischen Zahn und Zahnfleisch geleitet und abgestrahlt, was eine gleichmäßige, umfangreiche und tiefreichende Desinfektion bewirkt. Ferner ist ein und dasselbe flach aufgebaute Mundstück für eine Vielfalt an Gebissvariationen einsetzbar.

Die Strahlungsquellen können beliebige Strahlungsquellen bzw. Enden von mit entsprechender Strahlung gespeisten Wellenleitern sein. Besonders günstig ist, wenn die Strahlungsquellen Infrarot-LEDs sind. Diese sind effizient und für verschiedene Infrarot-Wellenlängenbereiche verfügbar, sodass eine Abstimmung auf den bzw. die verwendeten Photosensibilisator(en) einfach möglich ist. Überdies hat Infrarot-Strahlung zusätzlich eine wundheilende und entzündungshemmende Wirkung ("photobiologischer Effekt"). Die Infrarot-LEDs können beispielsweise LEDs für wassergefiltertes Infrarot-A ("wIRA-LEDs") sein.

Das Mundstück ist von einer zumindest im Wellenlängenbereich der Strahlungsquellen transparenten Beschichtung überzogen. Dies erhöht die Lebensdauer und die Anwendungssicherheit des Mundstücks und schützt die Zähne. Geeignete Beschichtungen sind langlebig, lichtecht, speichel- und abriebfest, schadstofffrei und dabei leicht aufzutragen, z.B. aufzuspritzen. Beispiele für solche Beschichtungen sind aus der Medizin, insbesondere der Zahnheilkunde, bekannt.

Ferner ist die Beschichtung über jeder Strahlungsquelle zu einer die Strahlung brechenden Linse geformt. Dadurch lässt sich die Strahlung leiten und mit nur wenigen Strahlungsquellen eine gleichmäßige Bestrahlung des gesamten Mundes bzw. der Zähne und des Zahnfleischs erzielen.

Vorteilhaft ist, wenn die Steuerung im Griffstück angeordnet ist. Dies erleichtert die Handhabung, da während der Verwendung der Bestrahlungsvorrichtung keine kabelgebundene oder kabellose Verbindung zur Steuerung erforderlich ist. Die Bestrahlungsvorrichtung kann dadurch platzsparend als integrierte Einheit aufgebaut werden.

Für den Benutzer besonders angenehm und in der Anwendung effektiv ist, wenn die Steuerung dazu ausgebildet ist, die Strahlungsquellen zum Abstrahlen zeitlich sich ändernder Strahlungsintensität anzusteuern.

Eine besonders effiziente Variante ergibt sich, wenn das Mundstück zumindest zwei Strahlungsquellen hat und die Steuerung dazu ausgebildet ist, die zumindest zwei Strahlungsquellen zum Abstrahlen voneinander verschiedener Strahlungsintensitäten anzusteuern.

In einer günstigen Ausführungsform hat die Bestrahlungsvorrichtung eine an Ober- und Unterseite des Mundstücks entlang der U-Form verlaufende Nut und eine in die Nut entnehmbar eingesetzte, zumindest im Wellenlängenbereich der Strahlungsquellen transparente Zahnschutzauflage. Dies verhindert ein unmittelbares Beißen auf empfindliche Teile des Mundstücks bzw. dessen Beschichtung, was die Vorrichtung schützt und ihre Verwendung noch sicherer macht.

Besonders vorteilhaft ist, wenn die Zahnschutzauflage aus Silikon ist. Silikon ist weich und dadurch besonders komfortabel in der Anwendung. Wird auf die Silikon-Zahnschutzauflage gebissen, haben das Mundstück und mit ihm die gesamte Bestrahlungsvorrichtung einen gleichbleibend sicheren Sitz im Mund; ferner wird durch das weiche und transparente Silikon die Einleitung der Strahlung in den Zahnschmelz verbessert, was die Wirksamkeit der Bestrahlung steigert.

Um eine möglichst gleichmäßige, anatomisch günstige Zahnauflage zu bieten, ist bevorzugt die Zahnschutzauflage in der Mitte der U-Form dicker als an den Enden der U-Form.

Gemäß einem zweiten Aspekt schafft die Erfindung ein System zur photodynamischen Desinfektion von Zähnen und Zahnfleisch in einem Mund, welches sich durch eine Bestrahlungsvorrichtung der vorgenannten Art und eine Spüllösung zum Spülen des Mundes in Vorbereitung einer Bestrahlung auszeichnet, welche Spüllösung zumindest einen Photosensibilisator enthält. Beim Spülen des Mundes mit der Spüllösung vor der Bestrahlung infiltriert der Photosensibilisator die Bakterien des Biofilms und verbleibt während der Bestrahlung im Mund, ohne dass die gesamte beim Spülen verwendete Spüllösung im Mund behalten werden müsste. Hinsichtlich weiterer Vorteile und Ausführungsformen des Systems wird auf die vorangegangenen Ausführungen zur Bestrahlungsvorrichtung verwiesen.

Die Spüllösung kann - neben dem Phtotosensibilisator - verschiedene Zusatzstoffe enthalten, welche die Desinfektionswirkung verstärken bzw. zusätzliche Wirkungen erzielen. In einer besonders vorteilhaften Ausführungsform enthält die Spüllösung einen Nano-Partikelträger, an welchen der Photosensibilisator gebunden ist. Besonders günstig ist dabei, wenn der Nano-Partikelträger ein Graphen-basierter Nano-Partikelträger oder Chitosan ist. Solche Nano-Partikelträger verbessern die Aufnahme des Photosensibilisators im Biofilm erheblich, was zu einer Verstärkung der Desinfektionswirkung führt. Ferner verbessern Nano-Partikelträger die Langzeitwirkung der Desinfektion, verhindern einen Rückgang der Bakterienempfindlichkeit und verringern mögliche Löslichkeitsprobleme bei gewissen Photosensibilisatoren, wodurch die Auswahl an einsetzbaren Photosensibilisatoren breiter wird.

Vorteilhaft ist, wenn die Spüllösung Kaliumiodid und/oder Urea enthält. Kaliumiodid und Urea verstärken jeweils die antimikrobielle photodynamische Wirkung der Spüllösung. Ebenfalls vorteilhaft ist, wenn die Spüllösung L-Arginin enthält. Diese Aminosäure wirkt als Biofilm-Disruptor und verstärkt dadurch auch die Wirkung des Photosensibilisators auf den Biofilm. Besonders güstig ist, wenn die Spüllösung Xylit enthält. Xylit blockiert den Stoffwechsel des Biofilms und beugt dadurch einer Neubildung von Biofilm zusätzlich vor. Günstig ist ferner, wenn die Spüllösung eine Nuklease, z.B. eine RNase oder DNase, enthält. Eine solche unterstützt den Abbau von durch die photodynamische Wirkung kompromittierten Bakterien im Biofilm.

Die Erfindung wird nachfolgend anhand eines in den beigeschlossenen Zeichnungen dargestellten Ausführungsbeispiels näher erläutert. Darin zeigt:
Fig. 1 eine erfindungsgemäße Bestrahlungsvorrichtung in ihrer in einen Mund eingeführten Stellung in einem vertikalen Längsschnitt;
Fig. 2 den Ausschnitt A von Fig. 1;
Fig. 3 die Bestrahlungsvorrichtung von Fig. 1 in einer Perspektivansicht von schräg oben; und
Fig. 4 die Bestrahlungsvorrichtung von Fig. 1 in der Draufsicht.

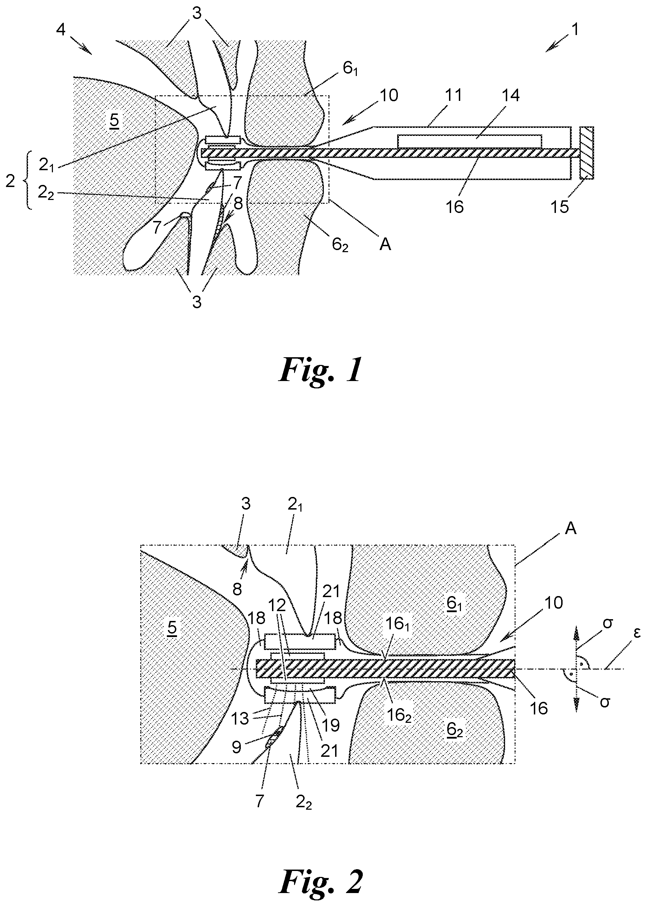

Fig. 1 zeigt eine Bestrahlungsvorrichtung 1 zur photodynamischen Desinfektion von Zähnen 2 (hier: oberen und unteren Schneidezähnen 2₁, 2₂) und Zahnfleisch 3 in einem Mund 4, der ausschnittsweise mit einer Zunge 5, einer Ober- und einer Unterlippe 6₁, 6₂ dargestellt ist. An dem unteren Schneidezahn 2₂ hat sich ein im Wesentlichen bakterieller Biofilm 7 gebildet. Auch an dem den unteren Schneidezahn 2₂ umgebenden Zahnfleisch 3 sowie in einer Zahnfleischfurche 8 zwischen dem unteren Schneidezahn 2₂ und dem Zahnfleisch 3 hat sich Biofilm 7 abgelagert. Solcher Biofilm 7 ist schädlich für Zähne 2₁, 2₂ und Zahnfleisch 3, da er unter anderem Karies, Gingivitis und/oder Parodontitis verursacht. Im Vergleich dazu ist der obere Schneidezahn 2₁ und das ihn umgebende Zahnfleisch 3 frei von Biofilm 7. Der schädliche Biofilm 7 soll durch photodynamische Desinfektion vernichtet werden, wie im Weiteren beschrieben.

Bei der photodynamischen Desinfektion wird ein Photosensibilisator (auch "Photosensitizer") in den Mund 4 eingebracht. Durch eine nachfolgende Bestrahlung wird der Photosensibilisator aktiviert, wobei der Photosensibilisator freie Radikale, z.B. reaktive Sauerstoffspezies ("ROS"), erzeugt, welche als Biozid den Biofilm 7 zerstören und dadurch die Zähne 2 und das Zahnfleisch 3 desinfizieren. Die meisten Photosensibilisatoren wirken als Katalysator, welcher bei Bestrahlung die freien Radikale aus der Umgebung erzeugt; andere Photosensibilisatoren geben bei Bestrahlung selbst freie Radikale ab.

Im vorliegenden Fall ist der Photosensibilisator 9 (Fig. 2) je nach Erfordernis z.B. Indocyaningrün, Methylenblau, Toluidinblau, Bengalrosa ("rose bengal"), Erythrosin, Acridinorange und/oder das zusätzlich antibiotisch wirkende Tetracyclin. Der Photosensibilisator 9 ist in einer Spüllösung enthalten, mit welcher der Benutzer in Vorbereitung der folgenden Bestrahlung seinen Mund 4 für einige Sekunden (z.B. etwa 30 Sekunden) spült. So wirken die Bestrahlungsvorrichtung 1 und die Spüllösung mit darin enthaltenen Photosensibilisator 9 als System zusammen. Es versteht sich, dass auch mehr als ein Photosensibilisator 9 in der Spüllösung enthalten sein kann.

Die Spüllösung ist beispielsweise auf Wasserbasis mit optional einem geringen Anteil (z.B. zwischen 0,5 und 1,5 Gew.-%, insbesondere 0,9 Gew.-%) an darin gelöstem Kochsalz hergestellt. Beim Spülen lagert sich der Photosensibilisator 9 an dem Biofilm 7 an und/oder infiltriert die Bakterien des Biofilms 7. Optional enthält die Spüllösung einen Nano-Partikelträger, z.B. einen Graphen-basierten Nano-Partikelträger, Chitosan etc., an welchen der Photosensibilisator gebunden ist und welcher von den Zellen des Biofilms 7 gut aufgenommen wird.

Die Spüllösung kann ferner optional Bakterizide und/oder Oxigenatoren etc. enthalten. Insbesondere enthält die Spüllösung optional Kaliumiodid, Urea, Zinnfluorid und/oder Aminfluorid zur Verstärkung der antimikrobiellen Wirkung, L-Arginin und/oder Methylsalicylat als Biofilm-Disruptor, Xylit zur Blockade des Stoffwechsels des Biofilms, eine Nuklease (z.B. eine RNase oder DNase) zum verbesserten Abbau von durch die photodynamische Wirkung kompromittierten Bakterien im Biofilm, Zinkchlorid zur leichteren Entfernung harter Beläge insbesondere an den Zähnen 2, Natriumfluorid zur Stärkung des Zahnschmelzes und/oder Flavonoide bzw. Catechine mit antioxidativer Wirkung.

Gemäß Fig. 1 umfasst die Bestrahlungsvorrichtung 1 ein Mundstück 10, welches in den Mund 4 eingeführt wird, und ein vom Mundstück 10 vorspringendes Griffstück 11, welches in der in den Mund 4 eingeführten Stellung des Mundstücks 10 aus dem Mund 4 herausragt. Das Mundstück 10 ist mit einer oder mehreren Strahlungsquellen 12 für Strahlung 13 in einem Wellenlängenbereich zwischen 500 nm und 1000 nm ausgestattet, d.h. die abgestrahlte Strahlung 13 liegt, je nach gewählter Type der Strahlungsquelle 13, in einem gewissen Wellenlängenbereich und hat ein ausgeprägtes Intensitätsmaximum zwischen 500 nm und 1000 nm. Die Strahlungsquellen 12 dienen zum Bestrahlen des mit Photosensibilisator 9 infiltrierten Biofilms 7 bzw. des Biofilms 7 mit angelagertem Photosensibilisator 9 und tragen dadurch über den photodynamischen Effekt hinaus - je nach Wellenlänge mehr oder weniger stark - zum photobiologischen Effekt, d.h. zur Wundheilung und Entzündungshemmung am Zahnfleisch 3, und somit zur Vorbeugung gegen Parodontitis bei. Die Bestrahlungsvorrichtung 1 hat ferner eine Steuerung 14, welche die Strahlungsquellen 12 ansteuert und im dargestellten Beispiel im Griffstück 11 angeordnet ist, alternativ jedoch separat davon und z.B. über ein Kabel mit den Strahlungsquellen 12 verbunden oder überhaupt im Mundstück 10 angeordnet sein könnte.

Das Griffstück 11 weist einen Kühlkörper 15 auf. In der Ausführungsform von Fig. 1 ist der Kühlkörper 15 eine Kühlrippe an dem dem Mundstück 10 abgewandten Ende des Griffstücks 11. In anderen Ausführungsformen kann der Kühlkörper 15 z.B. einen Satz von Kühlrippen haben bzw. ein Teil der Gehäuseoberfläche des Griffstücks 11 sein und dabei passiv, d.h. durch Konvektion, gekühlt sein. Alternativ kann der Kühlkörper aktiv gekühlt werden, z.B. mit einem Ventilator oder einem Peltierelement.

Die Strahlungsquellen 12 sind auf ein wärmeleitendes Substrat 16 aufgebracht. Das wärmeleitende Substrat 16 verbindet die Strahlungsquellen 12 des Mundstücks 10 mit dem Kühlkörper 15 des Griffstücks 11 wärmeleitend und erstreckt sich somit vom Mundstück 10 bis in das Griffstück 11.

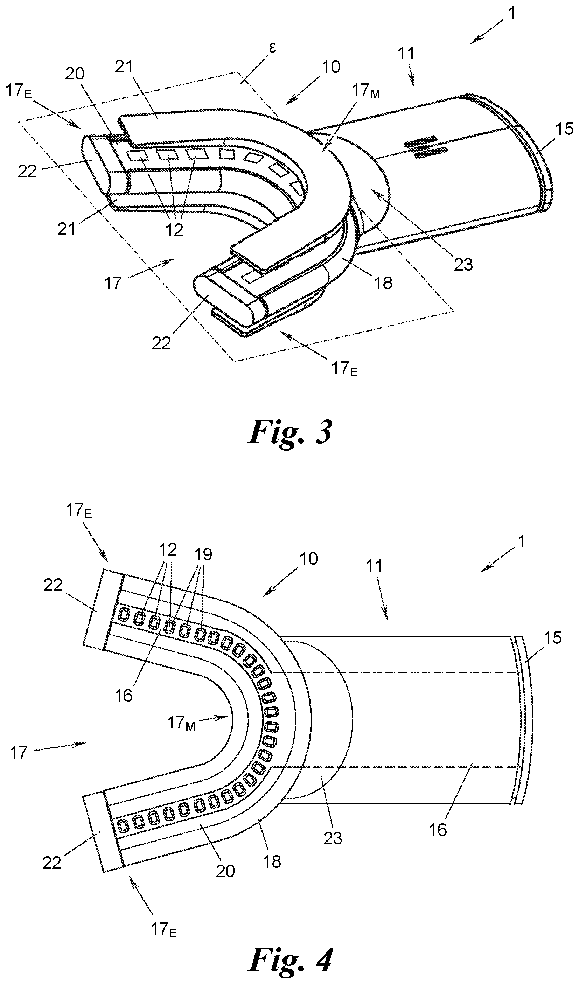

Wie in Fig. 3 gezeigt, hat das Mundstück 10 eine U-Form 17, welche dem Zahnbogen, z.B. einem durchschnittlichen Zahnbogen, nachgebildet ist. Die U-Form 17 spannt eine Ebene ε auf, welche in der in den Mund 4 eingeführten Stellung des Mundstücks 10 zwischen den Zähnen 2₁ des Oberkiefers einerseits und den Zähnen 2₂ des Unterkiefers andererseits liegt.

Das wärmeleitende Substrat 16 ist in dem dargestellten Beispiel eine in der Ebene ε liegende Metallkern-Leiterplatte, z.B. mit Aluminium- oder Kupferkern. Es versteht sich, dass das Substrat 16 nicht durchgehend eben sein muss, sondern z.B. eine oder mehrere Biegungen beispielsweise am Übergang vom Mundstück 10 zum Griffstück 11 und/oder im Griffstück 11 haben kann. Alternativ könnte das Substrat 16 beliebige Form haben oder auch eine Heatpipe sein.

Wie in Fig. 2 gezeigt, sind die Strahlungsquellen (z.B. LEDs, hier: Infrarot-LEDs, beispielsweise LEDs für wassergefiltertes Infrarot-A, "wIRA-LEDs") 12 optional sowohl an der Oberseite 16₁ als auch an der Unterseite 16₂ der Metallkern-Leiterplatte aufgebracht und haben dabei zu der genannten Ebene ε jeweils etwa normale Strahlungsrichtungen σ. Im vorliegenden Zusammenhang bezeichnet eine zu der Ebene ε normale Strahlungsrichtung σ eine unter der üblichen Divergenz einer Strahlungsquelle 12 von dieser abgestrahlte Strahlung 13, welche im Mittel normal zur Ebene ε gerichtet ist. Dabei können an Ober- und Unterseite 16₁, 16₂ jeweils unterschiedliche Anzahlen an Strahlungsquellen 12 aufgebracht sein. Alternativ dazu könnten die eine oder mehreren Strahlungsquellen 12 nur an der Oberseite 16₁ oder nur an der Unterseite 16₂ aufgebracht sein. Anstelle von (Infrarot-)LEDs können die Strahlungsquellen 12 anderer Art sein, z.B. herkömmliche (Infrarot-)Lampen. Ferner können Enden von mit der Strahlung 13 gespeisten Wellenleitern die Strahlungsquellen 12 bilden.

Jede Strahlungsquelle 12 hat dabei bevorzugt eine Leistungsdichte zwischen 0,5 J/cm² und 1000 J/cm². Die Wellenlänge der von jeder Strahlungsquelle 12 abgestrahlten Strahlung 13 liegt im Bereich von etwa 500 bis 1000 nm, bevorzugt im Rotlicht- oder Nah-Infrarotbereich ("NIR") zwischen 610 und 850 nm, und wird auf den Photosensibilisator 9, d.h. auf seine Absorptionswellenlänge, abgestimmt, bzw. wird umgekehrt der Photosensibilisator 9 in Abhängigkeit von der Wellenlänge der von der Strahlungsquelle 12 abgestrahlten Strahlung 13 gewählt; beispielsweise wird Indocyaningrün bei Bestrahlung unter einer Wellenlänge von etwa 810 nm aktiv, Methylenblau bei etwa 650 nm, Toluidinblau bei etwa 630 nm, Bengalrosa bei etwa 562 nm, Erythrosin bei etwa 530 nm, Acridinorange bei etwa 502 nm etc.

In dem dargestellten Beispiel der Fig. 1 bis 4 ist das Mundstück 10 von einer Beschichtung 18 überzogen, d.h. vollständig abgedeckt, welche die empfindlichen Teile des Mundstücks 10 vor Speichel und aggressiven Substanzen sowie vor mechanischer Abnutzung schützt. Umgekehrt schützt die Beschichtung 18 Zähne 2, Zunge 5 und Lippen 6₁, 6₂ vor Beschädigung bzw. Verletzung durch z.B. scharfkantige Teile des Mundstücks 10. Die Beschichtung 18 ist zumindest im Wellenlängenbereich des Intensitätsmaximums der von den Strahlungsquellen 12 abgestrahlten Strahlung 13, d.h. im Wellenlängenbereich der Strahlungsquellen 12, transparent und wird beispielsweise auf das Mundstück 10 aufgespritzt oder damit vergossen. Beschichtungen 18 dieser Art sind der Fachperson auf dem Gebiet der Medizin, insbesondere der Zahnheilkunde, bekannt.

Die Beschichtung 18 ist über jeder Strahlungsquelle 12, d.h. in Strahlungsrichtung σ jeder Strahlungsquelle 12, zu einer die Strahlung 13 brechenden Linse 19 geformt. Die Linse 19 ist je nach Bedarf konvex oder konkav gekrümmt, um die Strahlung 13 nach einem vorgegebenen Schema - z.B. gleichmäßig - über die Zähne 2 und das Zahnfleisch 3 im Mund 4 zu verteilen. Alternativ könnte die Linse 19 mit anderen Mitteln, d.h. nicht durch die Beschichtung 18 selbst, gebildet sein oder überhaupt entfallen.

Die Steuerung 14 ist optional dazu ausgebildet, die Strahlungsquellen 12 zum Abstrahlen zeitlich sich ändernder, z.B. pulsierender oder auf- und abschwellender, Strahlungsintensität anzusteuern. Die sich zeitlich ändernde Strahlungsintensität folgt dabei einem vorgegebenen Zyklus und ermöglicht hohe Spitzenintensität bei zugleich geringerer mittlerer Wärmeentwicklung im Mund 4. Insgesamt dauert die Bestrahlung zwischen 100 ms und 60 Minuten, z.B. etwa 2 bis 5 Minuten.

Zusätzlich oder alternativ kann die Steuerung 14, wenn das Mundstück 10 zumindest zwei Strahlungsquellen 12 hat, optional die Strahlungsquellen 12 zum Abstrahlen voneinander verschiedener Strahlungsintensitäten ansteuern. Im Beispiel der Fig. 1 könnte dazu beispielsweise die Steuerung 14 die auf den unteren Schneidezahn 2₂ gerichtete Strahlungsquelle 12 zum Abstrahlen einer hohen Strahlungsintensität ansteuern, da sich an dem und um den unteren Schneidezahn 2₂ schädlicher Biofilm 7 gebildet hat, wogegen die Steuerung 14 die auf den von Biofilm 7 freien oberen Schneidezahn 2₁ gerichtete Strahlungsquelle 12 zum Abstrahlen geringer Strahlungsintensität (hier: z.B. gar keiner Strahlung 13) ansteuert.

Im Beispiel der Fig. 3 hat das Mundstück 10 jeweils an seiner Ober- und Unterseite eine Nut 20, welche entlang der U-Form 17 des Mundstücks 10 verläuft. In die Nut 20 kann eine Zahnschutzauflage 21 eingesetzt werden. Die Zahnschutzauflage 21 ist zumindest im Wellenlängenbereich der Strahlungsquellen 12 transparent und optional wechselbar, d.h. sie kann nach dem Einsetzen in die Nut 20 wieder entnommen werden. Die Zahnschutzauflage 21 dient dem Schutz der Zähne 2 bzw. des Mundstücks 10, wenn z.B. während der Bestrahlung auf das Mundstück 10 gebissen wird. Dazu ist die Zahnschutzauflage 21 optional weich, z.B. aus Silikon gefertigt.

Um dem Mundstück 10 eine anatomisch geeignete Form zu geben, ist optional das Mundstück 10 selbst und/oder die Zahnschutzauflage 21 in der Mitte 17_{M} der U-Form 17 dicker als an den Enden 17_{E} der U-Form 17. Die Enden 17_{E} der U-Form 17 sind im Beispiel der Fig. 3 mit optionalen Kappen 22 verschlossen. Ferner hat zum besseren Schließen des Mundes 4 bei eingeführtem Mundstück 10 das Griffstück 11 optional ober- und/oder unterseitige Vertiefungen 23 im Anschluss an das Mundstück 10.

Die Erfindung ist nicht auf die dargestellte Ausführungsform beschränkt, sondern umfasst alle Varianten, Modifikationen und deren Kombinationen, die in den Rahmen der angeschlossenen Ansprüche fallen.

## Patentansprüche

1. Bestrahlungsvorrichtung zur photodynamischen Desinfektion von Zähnen (2) und Zahnfleisch (3) in einem Mund (4), umfassend
ein in den Mund (4) einführbares Mundstück (10), welches eine dem Zahnbogen nachgebildete U-Form (17) hat und mit einer oder mehreren Strahlungsquellen (12) für Strahlung (13) in einem Wellenlängenbereich zwischen 500 nm und 1000 nm ausgestattet ist,
ein Griffstück (11), welches vom Mundstück (10) derart vorspringt, dass es nach Einführen des Mundstücks (10) aus dem Mund (4) herausragt, und
eine Steuerung (14) zum Ansteuern der Strahlungsquellen (12),
wobei das Griffstück (11) einen Kühlkörper (15) aufweist und die Strahlungsquellen (12) auf ein wärmeleitendes Substrat (16) aufgebracht sind, welches die Strahlungsquellen (12) mit dem Kühlkörper (15) wärmeleitend verbindet, und wobei das Mundstück (10) von einer zumindest im Wellenlängenbereich der Strahlungsquellen (12) transparenten Beschichtung (18) überzogen ist,
**dadurch gekennzeichnet, dass**
die Beschichtung (18) über jeder Strahlungsquelle (12) zu einer die Strahlung (13) brechenden Linse (19) geformt ist.

2. Bestrahlungsvorrichtung nach Anspruch 1, **dadurch gekennzeichnet, dass** das Substrat (16) eine in einer durch die U-Form (17) aufgespannten Ebene (ε) liegende Metallkern-Leiterplatte ist.

3. Bestrahlungsvorrichtung nach Anspruch 2, **dadurch gekennzeichnet, dass** die Strahlungsquellen (12) auf zumindest eine der Ober- und Unterseiten (16₁, 16₂) der Metallkern-Leiterplatte aufgebracht sind und zur genannten Ebene (ε) normale Strahlungsrichtungen (σ) haben.

4. Bestrahlungsvorrichtung nach einem der Ansprüche 1 bis 3, **dadurch gekennzeichnet, dass** die Strahlungsquellen (12) Infrarot-LEDs sind.

5. Bestrahlungsvorrichtung nach einem der Ansprüche 1 bis 4, **dadurch gekennzeichnet, dass** die Steuerung (14) im Griffstück (11) angeordnet ist.

6. Bestrahlungsvorrichtung nach einem der Ansprüche 1 bis 5, **dadurch gekennzeichnet, dass** die Steuerung (14) dazu ausgebildet ist, die Strahlungsquellen (12) zum Abstrahlen zeitlich sich ändernder Strahlungsintensität anzusteuern.

7. Bestrahlungsvorrichtung nach einem der Ansprüche 1 bis 6, **dadurch gekennzeichnet, dass** das Mundstück (10) zumindest zwei Strahlungsquellen (12) hat und die Steuerung (14) dazu ausgebildet ist, die zumindest zwei Strahlungsquellen (12) zum Abstrahlen voneinander verschiedener Strahlungsintensitäten anzusteuern.

8. Bestrahlungsvorrichtung nach einem der Ansprüche 1 bis 7, **gekennzeichnet durch** eine an Ober- und Unterseite des Mundstücks (10) entlang der U-Form (17) verlaufende Nut (20) und eine in die Nut (20) entnehmbar eingesetzte, zumindest im Wellenlängenbereich der Strahlungsquellen (12) transparente Zahnschutzauflage (21).

9. Bestrahlungsvorrichtung nach Anspruch 8, **dadurch gekennzeichnet, dass** die Zahnschutzauflage (21) aus Silikon ist.

10. Bestrahlungsvorrichtung nach Anspruch 8 oder 9, **dadurch gekennzeichnet, dass** die Zahnschutzauflage (21) in der Mitte der U-Form (17) dicker als an den Enden der U-Form (17) ist.

11. System zur photodynamischen Desinfektion von Zähnen (2) und Zahnfleisch (3) in einem Mund (4), **gekennzeichnet durch** eine Bestrahlungsvorrichtung (1) nach einem der Ansprüche 1 bis 10 und eine Spüllösung zum Spülen des Mundes in Vorbereitung einer Bestrahlung, welche Spüllösung zumindest einen Photosensibilisator (9) enthält.

12. System nach Anspruch 11, **dadurch gekennzeichnet, dass** die Spüllösung einen Nano-Partikelträger, bevorzugt einen Graphen-basierten Nano-Partikelträger oder Chitosan, enthält, an welchen der Photosensibilisator (9) gebunden ist.

13. System nach Anspruch 11 oder 12, **dadurch gekennzeichnet, dass** die Spüllösung Kaliumiodid und/oder Urea enthält.

14. System nach einem der Ansprüche 11 bis 13, **dadurch gekennzeichnet, dass** die Spüllösung L-Arginin, Xylit und/oder eine Nuklease enthält.

## Claims

1. An irradiation device for photodynamic disinfection of teeth (2) and gums (3) in a mouth (4), comprising
a mouthpiece (10) which can be inserted into the mouth (4), has a U-shape (17) approximating the dental arch and is equipped with one or more radiation sources (12) for radiation (13) in a wavelength range between 500 nm and 1000 nm,
a handle piece (11) which projects from the mouthpiece (10) in such a way that it protrudes from the mouth (4) after insertion of the mouthpiece (10), and
a controller (14) for actuating the radiation sources (12),
wherein the handle piece (11) has a heat sink (15) and the radiation sources (12) are mounted on a heat-conducting substrate (16) which connects the radiation sources (12) to the heat sink (15) in a heat-conducting manner, and wherein the mouthpiece (10) is covered by a coating (18) which is transparent at least in the wavelength range of the radiation sources (12),
**characterised in that**,
above each radiation source (12), the coating (18) is formed into a lens (19) refracting the radiation (13).

2. The irradiation device according to claim 1, **characterised in that** the substrate (16) is a metal-core printed circuit board lying in a plane (ε) spanned by the U-shape (17).

3. The irradiation device according to claim 2, **characterised in that** the radiation sources (12) are mounted on at least one of the upper and lower sides (16₁, 16₂) of the metal-core printed circuit board and have radiation directions (σ) normal to said plane (ε).

4. The irradiation device according to any one of claims 1 to 3, **characterised in that** the radiation sources (12) are infrared LEDs.

5. The irradiation device according to any one of claims 1 to 4, **characterised in that** the controller (14) is arranged in the handle piece (11).

6. The irradiation device according to any one of claims 1 to 5, **characterised in that** the controller (14) is configured to actuate the radiation sources (12) for emitting radiation of a temporally changing radiation intensity.

7. The irradiation device according to any one of claims 1 to 6, **characterised in that** the mouthpiece (10) has at least two radiation sources (12) and the controller (14) is configured to actuate the at least two radiation sources (12) for emitting radiation having radiation intensities that are different from each other.

8. The irradiation device according to any one of claims 1 to 7, **characterised by** a groove (20) running along the U-shape (17) on the upper and lower sides of the mouthpiece (10) and by a tooth protection plate (21) which is removably inserted into the groove (20) and which is transparent at least in the wavelength range of the radiation sources (12).

9. The irradiation device according to claim 8, **characterised in that** the tooth protection plate (21) is made of silicone.

10. The irradiation device according to claim 8 or 9, **characterised in that** the tooth protection plate (21) is thicker in the middle portion of the U-shape (17) than at the end portions of the U-shape (17).

11. A system for photodynamic disinfection of teeth (2) and gums (3) in a mouth (4), **characterised by** an irradiation device (1) according to any one of claims 1 to 10 and a rinsing solution for rinsing the mouth in preparation for irradiation, which rinsing solution contains at least one photosensitiser (9).

12. The system according to claim 11, **characterised in that** the rinsing solution contains a nanoparticle carrier, preferably a graphene-based nanoparticle carrier or chitosan, to which the photosensitiser (9) is bound.

13. The system according to claim 11 or 12, **characterised in that** the rinsing solution contains potassium iodide and/or urea.

14. The system according to any one of claims 11 to 13, **characterised in that** the rinsing solution contains L-arginine, xylitol and/or a nuclease.

## Revendications

1. Dispositif d'irradiation pour la désinfection photodynamique des dents (2) et des gencives (3) dans une bouche (4), comprenant
un embout (10) pouvant être introduit dans la bouche (4), lequel a une forme en U (17) reproduisant l'arcade dentaire et est équipé d'une ou de plusieurs sources de rayonnement (12) pour un rayonnement (13) dans une plage de longueurs d'ondes entre 500 nm et 1000 nm,
une poignée (11) laquelle fait saillie hors de l'embout (10) de telle manière qu'elle dépasse de la bouche (4) après l'introduction de l'embout (10), et
une commande (14) pour l'actionnement des sources de rayonnement (12),
dans lequel la poignée (11) présente un dissipateur thermique (15) et les sources de rayonnement (12) sont appliquées sur un substrat (16) thermoconducteur, lequel relie les sources de rayonnement (12) avec le corps réfrigérant (15) en conduisant la chaleur, et où l'embout (10) est recouvert par un revêtement (18) transparent au moins dans la plage des longueurs d'onde des sources de rayonnement (12),
**caractérisé en ce que**
au-dessus de chaque source de rayonnement (12), le revêtement (18) est formé en une lentille (19) réfractant le rayonnement (13).

2. Dispositif d'irradiation selon la revendication 1, **caractérisé en ce que** le substrat (16) est une carte de circuits imprimés à noyau métallique se situant dans un plan (ε) tendu par la forme en U (17).

3. Dispositif d'irradiation selon la revendication 2, **caractérisé en ce que** les sources de rayonnement (12) sont appliquées sur au moins un des côtés supérieur et inférieur (16₁, 16₂) de la carte de circuits imprimés à noyau métallique et ont des directions de rayonnement (σ) normales par rapport audit plan (ε).

4. Dispositif d'irradiation selon l'une des revendications 1 à 3, **caractérisé en ce que** les sources de rayonnement (12) sont des DEL infrarouges.

5. Dispositif d'irradiation selon l'une des revendications 1 à 4, **caractérisé en ce que** la commande (14) est disposée dans la poignée (11).

6. Dispositif d'irradiation selon l'une des revendications 1 à 5, **caractérisé en ce que** la commande (14) est conçue pour actionner les sources de rayonnement (12) pour l'irradiation avec une intensité de rayonnement variant dans le temps.

7. Dispositif d'irradiation selon l'une des revendications 1 à 6, **caractérisé en ce que** l'embout (10) possède au moins deux sources de rayonnement (12) et la commande (14) est conçue pour actionner les au moins deux sources de rayonnement (12) pour l'irradiation avec des intensités de rayonnement différentes les unes des autres.

8. Dispositif d'irradiation selon l'une des revendications 1 à 7, **caractérisé par** une rainure (20) s'étendant sur la face supérieure et la face inférieure de l'embout (10) le long de la forme en U (17) et un protège-dents (21) transparent au moins dans la plage de longueurs d'ondes des sources de rayonnement (12) inséré de manière amovible dans la rainure (20).

9. Dispositif d'irradiation selon la revendication 8, **caractérisé en ce que** le protège-dents (21) est en silicone.

10. Dispositif d'irradiation selon la revendication 8 ou la revendication 9, **caractérisé en ce que** le protège-dents (21) est plus épais au milieu de la forme en U (17) qu'aux extrémités de la forme en U (17).

11. Système de désinfection photodynamique des dents (2) et des gencives (3) dans une bouche (4), **caractérisé par** un dispositif d'irradiation (1) selon l'une des revendications 1 à 10 et une solution de rinçage pour rincer la bouche en préparation d'une irradiation, laquelle solution de rinçage contient au moins un photosensibilisant (9).

12. Système selon la revendication 11, **caractérisé en ce que** la solution de rinçage contient un véhicule de nanoparticules, de préférence un véhicule de nanoparticules à base de graphène ou chitosane, sur lequel le photosensibilisant (9) est lié.

13. Système selon la revendication 11 ou 12, **caractérisé en ce que** la solution de rinçage contient de l'iodure de potassium et/ou de l'urée.

14. Système selon l'une des revendications 11 à 13, **caractérisé en ce que** la solution de rinçage contient de la L-arginine, du xylitol et/ou une nucléase.
